# EUROPEAN PATENT APPLICATION

(11) **EP 1 992 619 A1**
(43) Date of publication of application: **19.11.2008**
(21) Application number: 07380144.1
(22) Date of filing: 18.05.2007
(51) Int. Cl.: C07D 235/28, C07D 401/12

(54) **Process for preparing 2-(2-pyridylmethyl)-sulfinyl-1H-benzimidazoles and the intermediate compounds used therein**

(71) Applicant: Quimica Sintetica, S.A., 08028 Barcelona (ES)
(72) Inventor: Palomo Nicolau, Francisco Eugenio, 28804 Alcala de Henares (Madrid) (ES); Pastor del Castillo, Alfredo, 19005 Guadalajara (ES); Molina Ponce, Andrés, 19200 Azuqueca de Henares (Guadalajara) (ES)
(74) Representative: Barlocci, Anna

(57) **Abstract**

The present invention relates to a process for preparing 2-(2-pyridylmethyl)sulphinyl-1H-benzimidazoles that are proton pump inhibitors, using as intermediates 2-benzimidazolylsulphinic acid derivatives. The present invention also relates to said intermediate compounds, their use and a process for the preparation thereof.

These novel intermediate compounds are 2-benzimidazolylsulphinic acid esters that are obtained from their corresponding alkaline salts, which are in turn obtained by oxidation of substituted 2-mercaptobenzimidazoles.

The intermediate compounds of the invention are converted into 2-(2-pyridylmethyl)sulphinyl-1H-benzimidazoles by reaction with substituted 2-methylpyridines.

## Description

### Technical field

The present invention relates to a process for the preparation of compounds having a structure of 2-(2-pyridylmethyl)sulphinyl-1 H-benzimidazoles and that are used as antiulcer agents, to intermediate compounds used therein, and a method for their preparation.

### Background of the invention

Proton pump inhibitors (PPls) are a group of antiulcer agents having a structure of 2-(2-pyridylmethyl)sulphinyl-1H-benzimidazoles, and among them are included the compounds with the International Nonpropietary Name (INN) omeprazole, pantoprazole, lansoprazole, rabeprazole, and esomeprazole (S-enantiomer of omeprazole). The enantiomers of these compounds have also been described, i.e, R-omeprazole, S-pantoprazole, R-pantoprazole, S-lansoprazole, R-lansoprazole, S-rabeprazole, and R-rabeprazole. Usually, these antiulcer agents are used as pharmaceutically acceptable salts. For example, pantoprazole and rabeprazole are used in the form of sodium salt, and omeprazole and esomeprazol e in the form of magnesium salt.

Proton pump inhibitors are disclosed, for example, in EP-A-0005129, EP-A-0166287, EP-A-0174726, EP-A-0268956, and WO-A-96/02535.

The chemical structure of all these compounds contains a benzimidazole ring, a sulphoxide group (-SO-) and a pyridine ring:

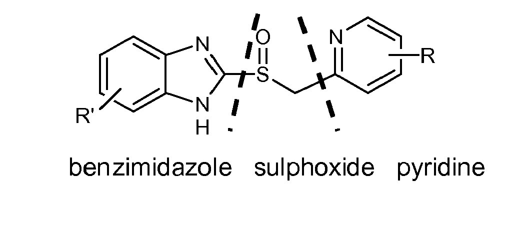

The process normally used for the preparation of these compounds includes a common final step that consists in generating the sulphoxide group by oxidation of the corresponding sulphide:

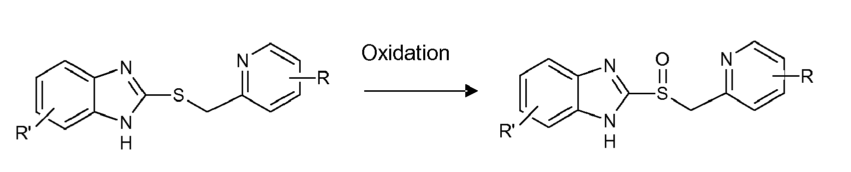

One of the drawbacks of this process lies in the fact that several overoxidized byproducts are generated in the oxidation step, for example, sulphone(-SO₂-) and N-oxide sulphoxide, which can only be removed after additional costly purification steps.

The difficulties involving oxidation are evidenced by the numerous alternatives that have been described in order to carry out the oxidation step. As an example, EP-A-0302720, EP-A-0484265, EP-A-0533264, EP-A-1071678, WO-A-91/18895, WO-A-99/25711, WO-A-02/062786, WO-A-03/008406, WO-A-2004/011455, WO-A-2004/018454, WO-A-2005/118569, WO-A-2006/074952, WO-A-2006/117802, WO-A-2007/017244, and WO-A-2007/026188 describe different alternative oxidation processes.

A different technical solution is the synthesis strategy described in other patent applications such as EP-A-1518857, WO-A-01/04109, WO-A-02/28852, WO-A-03/097606 and WO-A-2006/100243, where the sulphide group is not oxidized in the last step of the synthesis, but over an intermediate compound. In the case of pantoprazole, the oxidation reaction described therein is as follows:

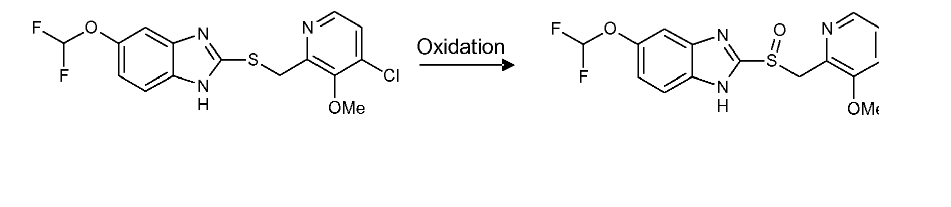

In line with such a strategy, pantoprazole is obtained in a subsequent step by means of a nucleophilic substitution of the chlorine atom in the 4 position of the pyridine ring by a methoxy group.

During these processes, impurities whose content depends on the reaction conditions (temperature, solvents,...) are also generated and must be removed by complex purification methods.

Generally, the presence of impurities, although in a small amount, makes the process scaling complicated, since it is more difficult to control impurities on an industrial scale, and t his implies that elaborate purification methods should be applied in order to obtain the products with the required purity.

Therefore, it is necessary that an improved process be available for the preparation of 2-(2-pyridylmethyl)sulphinyl-1H-benzimidazoles with a high yield and an appropriate purity, which would allow the products to be used as active ingredients in the preparation of pharmaceutical formulations.

### Summary of the invention

The authors of this invention have discovered a new process for preparing 2-(2-pyridylmethyl)sulphinyl-1 H-benzimidazoles, in which certain intermediate compounds having a sulphinate group are used. It should be emphasized that the new process prevents the formation of overoxidation impurities from the sulphide group, such as sulphones and N-oxide sulphoxides derivatives, and the benzimidazole derivatives can be obtained with a high yield.

Therefore, an object of the invention is a new process for the preparation of 2-(2-pyridylmethyl)sulphinyl-1H-benzimidazoles and their pharmaceutically acceptable salts starting from easily obtainable intermediate compounds.

Another object of this invention relates to new intermediate compounds for the preparation of 2-(2-pyridylmethyl)sulphinyl-1 H-benzimidazoles, as well as a process for the preparation of said intermediate compounds which are 2-benzimidazolylsulphinic acid derivatives.

Another object of the invention is the use of these intermediate compounds in the preparation of 2-(2-pyridylmethyl)sulphinyl-1 H-benzimidazoles and their pharmaceutically acceptable salts.

### Detailed description of the invention

### Preparation of 2-(2-pyridylmethyl)sulphinyl-1H-benzimidazoles, compounds of general formula (II)

An object of the invention is a process for preparing the compounds of general formula (II) 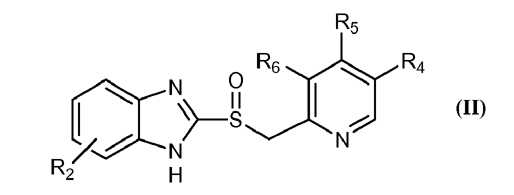 wherein:
R₂ is hydrogen, C₁-C₄ alkyl, C₁-C₄ alkoxy, C₁-C₄ alkoxy partially or totally substituted with halogen atoms,
R₄, R₅ and R₆, independently represent hydrogen, C₁-C₈ alkyl, C₁-C₈ alkoxy, C₁-C₈ alkoxy partially or totally substituted with alkoxide groups, C₁-C₈ alkoxy partially or totally substituted with halogen atoms,
characterized in that it comprises the reaction of a compound of general formula (I): 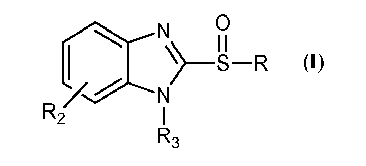 wherein:
R₂ is hydrogen, C₁-C₄ alkyl, C₁-C₄ alkoxy, C₁-C₄ alkoxy partially or totally substituted with halogen atoms, and
R is -OR₁, wherein R₁ is C₁-C₈ alkyl, C₂-C₈ alkenyl, C₃-C₂₀, cycloalkyl or C₇-C₂₀ alkylaryl,
R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, carbonyloxyalkyl or silylalkyl,
with a metalated derivative of the compound of formula (III): 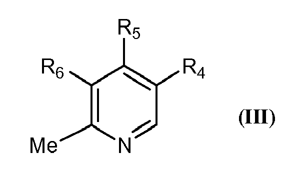 wherein:
R₄, R₅ and R₆, independently represent hydrogen, C₁-C₈ alkyl, C₁-C₈ alkoxy, C₁-C₈ alkoxy partially or totally substituted with alkoxide groups, C₁-C₈ alkoxy partially or totally substituted with halogen atoms,
in the core of an inert solvent.

Preferably the compounds have the general formula (II) wherein R₂ is hydrogen, C₁-C₃ alkoxy or C ₁-C₃ alkoxy partially or totally substituted with fluor atoms, more preferably R₂ is hydrogen, methoxy, or difluoromethoxy.

Preferably the compound of general formula (II) is omeprazole, pantoprazole, lansoprazole or rabeprazole.

The starting material is a compound of general formula (I) as mentioned above.

Preferably the compounds have the general formula (I) wherein R₂ is hydrogen, C₁-C₃ alkoxy or C₁-C₃ alkoxy partially or totally substituted with fluor atoms, R is -OR₁ wherein R₁ is C₁-C₄ alkyl, C₃-C₁₂ cycloalkyl, or C₇-C₁₆, alkylaryl and R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl.

More preferably R₂ is hydrogen, methoxy, or difluorom ethoxy, and R is -OR₁ wherein R₁ is methyl, ethyl, *n*-propyl, *i*-propyl, (-)-menthyl, (+)-menthyl, (-)-fenchyl, (+)-fenchyl, (-)-8-phenylmenthyl, (+)-8-phenylmenthyl, and R₃ is hydrogen, or alkali or alkaline earth metal cation.

Yet more preferably, R₂ is hydrogen, methoxy, or difluoromethoxy, and R is -OR₁ wherein R₁ is ethyl, (-)-menthyl, or (-)-fenchyl.

The metalated derivatives of the compounds of formula (III) are metalated in the methyl group adjacent to the nitrogen atom of the pyridine ring. These derivatives may be obtained either from substituted 2-methylpyridines, which are described for example in EP-A-0005129, EP-A-0166287, EP-A-0174726, or EP-A-0268956, or from halomethyl derivatives of the substituted 2-methylpyridines by metalation reactions according to methods well known in the art.

Preferably, the metalated derivative of compound (III) is a compound of formula (VI) 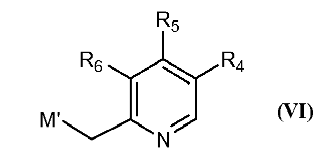 wherein:
R₄, R₅ and R₆ are as defined above, and
M' is an alkali or alkaline earth metal cation, a Mg-halogen cation, or a Zn-halogen cation.

In the compounds of formula (VI), M' is preferably a lithium cation.

In a preferred embodiment, the comp ound of formula (III) is selected from the group consisting of compounds wherein R₄ is hydrogen or methyl, R₅ is methoxy, methyloxypropyloxy, or 2,2,2-trifluoroethoxy, and R₆ is methyl or methoxy.

More preferably the compounds of formula (III) are selected from the group consisting of compounds wherein R₄ is hydrogen and R₅ and R₆ are methoxy; R₄ is hydrogen, R₅ is methyloxypropyloxy and R₆ is methyl; R₄ is methyl, R₅ is methoxy and R₆ is methyl; and R₄ is hydrogen, R₅ is 2,2,2-trifluoroethoxy, and R₆ is methyl.

The reaction between the compound of general formula (I) and the metalated derivative of the compound of formula (III) is carried out in the core of an inert solvent, for example, anhydrous tetrahydrofuran or anhydrous ethyl ether. Preferably, anhydrous tetrahydrofuran is used.

The matalation reaction for preparing the compound of formula (VI) may be carried out, for example, with *n*-butyl lithium at low temperature, in general, below -80°C in an inert solvent such as anhydrous tetrahydrofuran.

Generally, the metalated derivative of the compound of formula (III) is not isolated, and the solution obtained is slowly added to a solution of the compound of general formula (I) in an inert solvent such as tetrahydrofuran, cooled to a low temperature normally below -80°C.. During the addition, the solution is usually maintained at low temperature, for example, below -80°C.

Thereafter, the resulting mixture is generally allowed to stand until reaching a temperature of approximately -20°C and then is allowed to reach room temperature after adding water.

The compound of general formula (II) is isolated using conventional methods.

Surprisingly, it has been observed that by using the process of the present invention, 2-(2-pyridylmethyl)sulphinyl-1H-benzimidazoles can be prepared with a high degree of purity, since its use prevents the formation of impurities that can hardly be removed, such as sulphone and N-oxide sulphoxide derivatives, which are normally formed during the processes described for the preparation of said compounds. This fact contributes to avoiding complex purification methods and allows obtaining these compounds with a high yield.

Consequently, benzimidazole derivatives including omeprazole, pantoprazole, lansoprazole, and rabeprazole may be prepared by the process of the invention with a high yield.

### Preparation of 2-(2-pyridylmethyl)sulphinyl-1H-benzimidazole enantiomers, compounds of general formula (II)

2-(2-pyridylmethyl)sulphinyl-1H-benzimidazoles, compounds of general formula (II), have a sulphur atom bound to four different groups (where the pair of unshared electrons is considered to be another group, necessarily different from the others) and thus, optical activity may be present.

Although proton pump inhibitors such as pantoprazole or omeprazole are, in fact, normally used in racemic form in pharmaceutical formulations, pure enantiomers may also be used, for example, esomeprazole which is the INN of S-enantiomer of omeprazole.

Accordingly, one of the advantages of the process of the invention consists in the fact that each enantiomer of 2-(2-pyridylmethyl)sulphinyl-1 H-benzimidazoles may be prepared if the R group of the compound of general formula (I) is -OR₁ wherein R₁ is a chiral alcohol radical such as (-)-menthol, (+)-menthol, (-)-fenchol, (+)-fenchol, (-)-8-fenylmentol, (+)-8-fenylmentol. Thereafter, the optical isomers of the compound of general formula (I) are separated by conventional methods.

The compound of general formula (I) is formed by a mixture of optical isomers.

This is due to the fact that the sulphur atom, which is contained in the compounds of general formula (I), is bound to three different atom groups and has a pair of free electrons. This structure may result in an optical activity as it happens when a carbon atom is bound to four different groups, since this sulphur atom is also bound to four different groups.

When the compound of general formula (I) contains a chiral alcohol radical in the R group, a mixture of optical isomers, which are diastereoisomers, is obtained, i.e. optical isomers that are not enantiomers. Enantiomers are those optical isomers, one of which is the mirror image of the other.

This is due to the fact that although the sulphur atom of the compound of general formula (I) may exhibit two possible configurations, the chiral alcohol exhibits only one of them.

The methods that can be used in the separation of mixtures formed by diastereoisomers are well known by a person skilled in the art.

Unlike the enantiomers, the diastereoisomers normally show sufficiently different physical properties to be separated. For example, different solubilities in a same solvent.

One of the methods that can be used for separating the optical isomers of the compound of general formula (I) consists of the use of a column for high-performance liquid chromatography designed for the separation of diastereoisomers, e.g., DISCOVERY ZR-CARBON column series supplied by Sigma-Aldrich.

In a preferred embodiment, the preparation of the compound of general formula (II) by reaction between the compound of general formula (I) and the compound of formula (III) comprises a previous step, which comprises the separation of the optical isomers of the compound of general formula (I) wherein R₁ is a radical corresponding to a chiral alcohol.

Chiral alcohols, which may be used in the process of the invention, may be selected , for example, from the groups consisting of (-)-menthol, (+)-menthol, (-)-fenchol, (+)-fenchol, (-)-8-phenylmenthol, and (+)-8-phenylmenthol. Preferably, (-)-menthol or (-)-fenchol are used.

On carrying out the reaction between the compound of formula (III) and one of the optical isomers of the compound of general formula (I), one of the optical isomers of the compound of general formula (II) can be obtained in a stereospecifc form. Consequently, S-omeprazole, R-omeprazole, S-pantoprazole, R-pantoprazole, S-lansoprazole, R-lansoprazole S-rabeprazole, and R-rabeprazole may be obtained by means of the process of the invention.

### Preparation of the compounds of general formula (I)

It is an object of the invention a process for the preparation of the compounds of general formula (1) 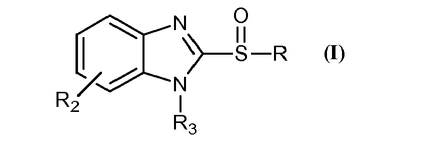 wherein:
R₂ is hydrogen, C₁-C₄ alkyl, C₁-C₄ alkoxy, C₁-C₄ alkoxy partially or totally substituted with halogen atoms, and
R is -OR₁, wherein R₁ is C₁-C₈ alkyl, C₂-C₈ alkenyl, C₃-C₂₀ cycloalkyl or C₇-C₂₀ alkylaryl,
R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl,
characterized in that it comprises reacting a compound of general formula (IV) 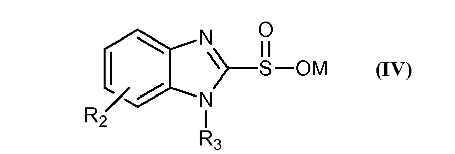 wherein:
R₂ is hydrogen, C₁-C₄ alkyl, C₁-C₄ alkoxy, C₁-C₄ alkoxy partially or totally substituted with halogen atoms,
M is an alkali or alkaline earth metal cation, and
R₃ is hydrogen, alkali or alkaline earth metal cation, C₁-C₄, alkyl, C₁-C₄ alkyl partially or totally substituted with halogen atoms, alkenyl, sulfonylalkyl, sulfonylamino, carbonylalkyl, cabonyloxyalkyl or silylalkyl,
with a C₁-C₈ alkyl, C₂-C₈ alkenyl, C₃-C₂₀ cycloalkyl o r C₇-C₂₀ alkylaryl alcohol by previously converting the -OM group of compound (IV) into a leaving group.

Preferably, a compound of general formula (I), wherein R₂ is hydrogen, C₁-C₃ alkoxy or C₁-C₃ alkoxy partially or totally substituted with fluor atoms, R is -OR₁ wherein R₁ is C₁-C₄ alkyl, C₃-C₁₂ cycloalkyl, or C₇-C₁₆, alkylaryl and R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl, is obtained.

More preferably, R₂ is hydrogen, methoxy, or difluoromet hoxy, and R is -OR₁ wherein R₁ is methyl, ethyl, *n*-propyl, *i*-propyl, (-)-menthyl, (+)-menthyl, (-)-fenchil, (+)-fenchil, (-)-8-phenylmenthyl, (+)-8-phenylmenthyl, and R₃ is hydrogen.

Yet more preferably, R₂ is hydrogen, methoxy, or difluoromethoxy, and R is -OR₁ wherein R₁ is ethyl, (-)-menthyl, or (-)-fenchyl.

Group R₃ in the compound of general formula (I) may also be any protective group which is used for the protection of the amino group such as, for example, those protective groups reported by T. W. Greene et al., in "Protective Groups in Organic Synthesis", 3rd Edition, John Wiley & Sons, New York, 1999 [ISBN: 0-471-16019-9].

Preferably, the starting material is a compound of general formula (IV) wherein R₂ is hydrogen, C₁-C₃ alkoxy or C₁-C₃ alkoxy totally or partially substituted with fluor atoms, R₃ is hydrogen or an alkali or alkaline earth metal cation, C₁-C₄ alkyl, C₁-C₄ alkyl partially or totally substituted with halogen atoms, alkenyl, sulfonylalkyl, sulfonylamino, carbonylalkyl, carbonyloxyalkyl or silylalkyl, and M is a alkali or alkaline earth metal cation.

More preferably, R₂ is hydrogen, C₁-C₃ alkoxy o r C ₁-C₃ alkoxy totally or partially substituted with fluor atoms, and R₃ and M are an alkali or alkaline earth metal cation, and even more preferably R₂ is hydrogen, methoxy, or difluoromethoxy, and R₃ and M are a sodium or potassium cation.

In the process of the invention, the -OM group of the compound of the general formula (IV) is previously converted into a leaving group.

This customary technique is used, for example, for the preparation of carboxylic acid esters starting from the same acids, in which the -OH group is converted into a leaving group that can react with an alcohol. Among the techniques used thereby, the following ones are underlined: preparation of a mixed anhydride by reaction with carboxylic acid halides or with alkyl- or arylsulfonic acid halides, reaction with dicyclohexylcarbodiimide or N,N' -carbonyldiimidazole, reaction with ethyl azodicarboxylate (Mitsunobu esterification), reaction with dialkylhalophosphates, reaction with triflic anhydride, reaction with oxazolidinones of phosphinic acid halides, or the reaction with alkoxycarbonyl halides.

Preferably, the -OM group of the compound of general formula (IV) is converted into a leaving group by reaction of this compound with a compound selected from group consisting of oxazolidinones of phosphinic acid halides, alkoxycarbonyl halides, carboxylic acid halides, alkylcarbodiimides or N,N'-carbonyldiimidazole, more preferably the compound of the general formula (IV) is reacted with a C₂-C₆ carboxylic acid chloride, more preferably with pivalic acid chloride.

The conversion of the -OM group into a leaving group activates the sulphinate group of the compound of general formula (I) for the subsequent reaction with the alcohol.

Alkylcarbodiimides, include, for instance, N'-dicyclohexylcarbodiimide.

Oxazolidinones of phosphinic acid halides include, for instance, bis(2-oxo-3-oxazolidinyl)phosphinic acid chloride, which is commercially available and supplied, for example, by Aldrich.

Alkoxycarbonyl halides include, for instance, ethyl chloroformiate.

Generally, the activation reaction of the sulphinate group undergoes in the core of an inert solvent. Preferably, a solvent selected from the group consisting of toluene, acetonitrile, diclhoromethane, and chloroform is used, more preferably dichloromethane is used.

The previous activation reaction may occur at a temperature ranging from -20°C to 20°C, preferably the reaction occurs at a temperature ranging from -10°C to 0° C.

In the reaction with acid chloride, catalysts may be used such as for example 4-picoline or 4-dimethylaminopiridine.

Preferably, a molar excess of acid chloride is used versus starting material, i.e., the compound of general formula (IV). More preferably the molar excess ranges from 1.05 to 2.5, and even more preferably from 1.75 to 2.0.

The subsequent reaction with an alcohol is usually carried out without isolating the activated intermediate, thus avoiding an isolation step with the consequent yield loss.

The reaction with the alcohol is advantageously carried out in the core of the same inert solvent used.

The alcohol used to carry out this reaction is a C₁-C₈ alkyl, C₂-C₈ alkenyl, C₃-C₂₀ cycloalkyl or C₇-C₂₀ alkylarylic alcohol.

Preferably the alcohol is a C₁-C₄ alkyl, C₃-C₁₂ cycloalkyl or C₇-C₁₆ alkylaryl alcohol, more preferably the alcohol is methanol, ethanol, *n*-propanol, *i*-propanol, (-)-menthol, (+)-menthol, (-)-fenchol, (+)-fenchol, (-)-8-phenylmenthol or (+)-8-phenylmenthol.

As discussed above the use of a chiral alcohol, such as (-)-menthol, (+)-menthol, (-)-fenchol, (+)-fenchol, (-)-8-phenylmenthol, (+)-8-phenylmenthol, shows some advantages, since it makes easier the separation of the optical isomers of the compound of general (I) and, consequently, the preparation of optically active 2-(2-pyridylmethyl)sulinyl-1H-benzimidazole isomers (enantiomers), such as S-omeprazole, which INN is esomeprazole.

The reaction with the alcohol may be carried out at a temperature ranging from -10° C to 5°C, preferably below 0°C.

Preferably, almost equivalent amounts of alcohol versus starting material, the compound of general formula (IV), are used, i.e., from 1.0 to 1.2 alcohol equivalents.

The a Icohol may be slowly added to a cold solution of the intermediate obtained. Generally, the same inert solvent (e.g. methylene chloride) used in the first reaction step is added as a solution.

After completion of addition, the reaction mass is allowed to stand until reaching room temperature generally over a period of 1 and 3 hours.

The reaction product is treated by conventional methods for separation of the formed salts, for example, by adding water and subsequent separation of phases. In general, pH of aqueous phase is adjusted to a range from 7 to 9.

The compound of general formula (I) is obtained by evaporation of the organic phase and may be purified by methods known by a person skilled in the art, such as for example by recrystallization or column chromatography.

### Preparation of the compounds of general formula (IV)

In a preferred embodiment, the compound of general formula (IV) 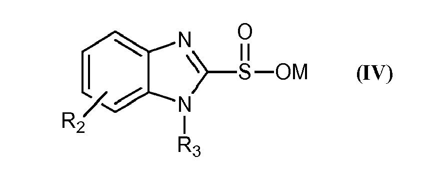 wherein:
R₂ is hydrogen, C₁-C₄ alkyl, C₁-C₄ alkoxy, C₁-C₄ alkoxy partially or totally substituted with halogen atoms,
M is hydrogen, or an alkali or alkaline earth metal cation, and
R₃ is hydrogen, an alkali or alkaline earth metal cation, C₁-C₄ alkyl, C₁-C₄ alkyl partially or totally substituted with halogen atoms, alkenyl, sulfonylalkyl, sulfonylamino, carbonylalkyl, carbonyloxyalkyl or silylalkyl,
is obtained by a process which comprises reactin g a compound of general formula (V): 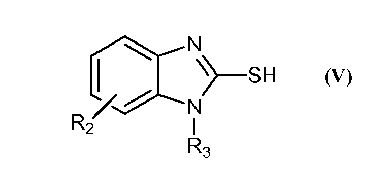 wherein:
R₂ is hydrogen, C₁-C₄ alkyl. C₁-C₄ alkoxy, C₁-C₄ alkoxy partially or totally substituted with halogen atoms, and
R₃ is hydrogen, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl,
with an oxidative agent in an alkaline medium.

Preferably the obtained product is a compound of the general formula (IV) wherein R₂ is hydrogen, C₁-C₃ alkoxy, or C₁-C₃ alkoxy totally or partially substituted with fluor atoms, M is hydrogen or an alkali or alkaline earth metal cation, and R₃ is hydrogen or an alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl.

More prefer ably R₂ is hydrogen, C₁-C₃ alkoxy or C ₁-C₃ alkoxy totally or partially substituted with fluor atoms, and R₃ and M are an alkali or alkaline earth metal cation, and even more preferably R₂ is hydrogen, methoxy, or difluoromethoxy, and R₃ and M are a sodium or potassium cation.

The oxidation may be carried out in the presence of peracids, such as for example *m*-chloroperbenzoic acid, or alkyl hydroperoxides such as *t*-butyl peroxide , or hydrogen peroxide and cat alysis of molybdate salts. Preferably hydrogen peroxide and catalysis of molybdate salts are used, and more preferably hydrogen peroxide and ammonium heptamolybdate are used.

The oxidation reaction is carried out in an alkaline medium. The pH of the alkaline medium is basic, i.e., greater than 7. Thus, the reaction occurs preferentially on the core in an aqueous solution of an alkali or alkaline earth hydroxide, and more preferably sodium hydroxide.

In order to carry out the oxidation reaction , for example, using hydrogen peroxide and ammonium heptamolybdate, generally the starting material of general formula (V) is dissolved in the core of an aqueous alkali hydroxide solution, and then the mixture is cooled at a temperature ranging from -20° C to 20° C, preferably from -10°C to 10°C. The concentration of the aqueous alkali hydroxide solution preferably ranges from 0.1 N to 10 N, more preferably from 0.5 N to 2 N, and even more preferably 1 N approximately.

To this solution, ammonium heptamolybdate is added, the temperature is maintained below 0°C, and hydrogen peroxide is slowly added. Once the addition is completed, the resulting mixture is allowed to stand until reaching room temperature.

For product isolation, the solvent may be removed under reduced pressure, and water traces may be eliminated from the product by methods well known in the art, for example, azeotropic distillation with toluene.

The obtained product is sufficiently pure to be used directly as starting material for the preparation of the compound of general formula (I).

If desired, the product may be later purified, for example, by esterification with an alcohol and subsequent akaline hydrolysis.

### Intermediate compounds

The inven tion encompasses the intermediate compounds of general formula (I) 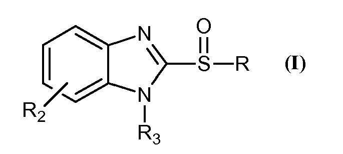 wherein:
R₂ is hydrogen, C₁-C₄ alkyl, C₁-C₄ alkoxy, C₁-C₄ alkoxy partially or totally substituted with halogen atoms, and
R is -OR₁, wherein R₁ is hydrogen, an alkali or alkaline earth metal cation, C₁-C₈ alkyl, C₂-C₈ alkenyl, C₃-C₂₀ cycloalkyl or C₇-C₂₀ alkylaryl,
R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxialkyl or silylalkyl,
and any of their optical isomers, with the exception of the compound wherein R₁, R₂, and R₃ are hydrogen.

The compound of general formula (I) wherein R₂, R₃ and R₁ are hydrogen is explicitly excluded from the invention because it is described in Abramova et al., Khimiya Geterotsiklicheskikh Soedinenii, 1975, 12, 1674-1677, Willson et al., Eur. J. Med. Chem., 1989, 24, 623-625, and Willson et al., Eur. J. Med. Chem., 1992, 27, 799-808.

Preferably the compounds have the general formula (I) wherein R₂ is hydrogen, C₁-C₃ alkoxy o r C ₁-C₃ alkoxy totally or partially substituted with fluor atoms, R is -OR₁ wherein R₁ is C₁-C₄ alkyl, C₃-C₁₂ cycloalkyl or C₇-C₁₆ alkylaryl and R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl o r sil ylalkyl. More preferably R₂ is hydrogen, methoxy, or difluoromethoxy, R₁ is methyl, ethyl, *n-*propyl, *i*-propyl, (-)-menthyl, (+)-menthyl, (-)-fenchil, (+)-fenchil, (-)-8-phenylmenthyl, (+)-8-phenylmenthyl, and R₃ is hydrogen. Yet more preferably, R₂ is hydrogen, methoxy, or difluoromethoxy, and R₁ is ethyl, (-)-menthyl, or (-)-fenchil.

The R₃ group in the compound of general formula (I) may also be any protective group that is used for the protection of the amino group, such as for example those mentioned in Greene's publication (see above).

Preferably the compound of the invention is a compound of the general formula (I) wherein R₂ is hydrogen, C₁-C₃ alkoxy o r C ₁-C₃ alkoxy totally or partially substituted with fluor atoms, R is -OR₁, wherein R₁ is an alkali or alkaline earth metal cation and R₃ is hydrogen or an alkali or alkaline earth metal cation. More preferably R₂ is hydrogen, methoxy, or difluoromethoxy, and R₁ and R ₃ are an alkali metal cation, and even more preferably R₂ is hydrogen, methoxy, or difluoromethoxy, and R₁ and R₃ are a sodium cation or a potassium cation.

### Use of the compounds of general formula (I)

It is also an object of the invention the use of compounds derived from 2-benzimidazolylsulphinic acid for the preparation of 2-(2-pyridylmethyl)sulphinyl-1 H-benzimidazoles and their pharmaceutically acceptable salts.

Preferably 2-benzimidazolylsulphinic acid derivatives are represented by the general formula (I) 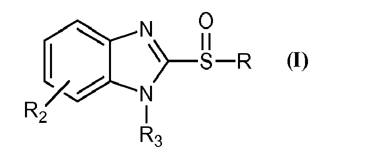 wherein:
R₂ is hydrogen, C₁-C₄ alkyl, C₁-C₄ alkoxy, C₁-C₄ alkoxy partially or totally substituted with halogen atoms, and
R is -OR₁, wherein R₁ is hydrogen, a alkali or alkaline earth metal cation, C₁-C₈ alkyl, C₂-C₈ alkenyl, C₃-C₂₀ cycloalkyl or C₇-C₂₀ alkylaryl,
R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl.

Preferably the compounds have the general formula (I) wherein R₂ is hydrogen, C₁-C₃ alkoxy or C₁-C₃ alkoxy totally or partially substituted with fluor atoms, R is -OR₁ wherein R₁ is C₁-C₄ alkyl, C₃-C₁₂ cycloalkyl or C₇-C₁₆ alkylaryl and R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or sil ylalkyl. More preferably, R₂ is hydrogen, methoxy, or difluoromethoxy, R₁ is methyl, ethyl, *n-*propyl, *i*-propyl, (-)-menthyl, (+)-menthyl, (-)-fenchil, (+)-fenchil, (-)-8-phenylmenthyl, (+)-8-phenylmenthyl, and R₃ is hydrogen or alkali or alkaline earth metal cation. Yet more preferably, R₂ is hydrogen, methoxy, or difluoromethoxy, and R₁ is ethyl, (-)-menthyl, or (-)-fenchil.

Preferably the compound of the invention is a compound of general formula (I) wherein R₂ is hydrogen, C₁-C₃ alkoxy or C ₁-C₃ alkoxy totally or partially substituted with fluor atoms, R is -OR₁, wherein R₁ is a alkali or alkaline earth metal cation, and R₃ is hydrogen or an alkali or alkaline earth metal cation. More preferably R₂ is hydrogen, methoxy, or difluoromet hoxy, and R₁ and R ₃ are an alkali metal cation, and even more preferably R₂ is hydrogen, methoxy, or difluoromethoxy, and R₁ and R₃ are a sodium cation or a potassium cation.

Preferably the 2-(2-pyridylmethyl)sulphinyl-1H-benzimidazole derivatives that are prepared from the compounds of general formula (I) are omeprazole, pantoprazole, lansoprazole, rabeprazole, esomeprazole (S-omeprazole), R-omeprazole, S-pantoprazole, R-pantoprazole, S-lansoprazole, R-lansoprazole, S-rabeprazole, and R-rabeprazole.

### Industrial application

The 2-(2-pyridylmethyl)sulphinyl-1H-benzimidazole derivatives obtained by the process of the invention may be used for the formulation of antiulcer drugs such as those described, for example, in EP-A-0005129, EP-A-0166287, EP-A-0174726, and EP-A-0268956.

The following non-limiting examples illustrate the present invention.

### Examples

### Example 1.- Preparation of 5-methoxy-2-benzimidazolylsulphinic acid disodium salt, compound of general formula (IV) wherein R₂ is 5-methoxy, and M and R₃ are Na⁺

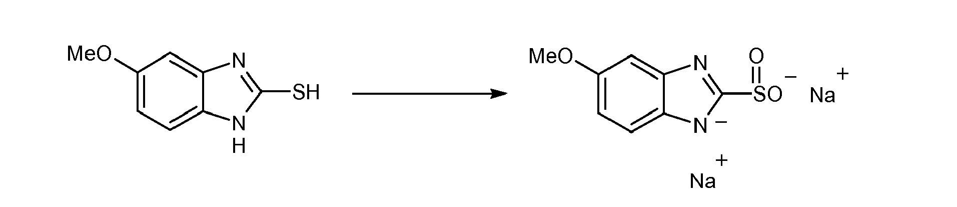

54.07 g (0.3 mol) of 2-mercapto-5-methoxybenzimidazole in 0.75 I of 1 N sodium hydroxide (0.75 mol) were dissolved at room temperature, and the mixture was cooled below -5° C.

2-mercapto-5-methoxybenzimidazole may be prepared in accordance with the method described in EP-A-0005129 or may be obtained from commercial sources (Aldrich).

3.71g (3 mmol) of ammonium heptamolybdate tetrahydrate were added and, at a sustained temperature of below 0° C, 61.24 g (0.63 mol) of 35% hydrogen peroxide solution were slowly added.

Once the addition was completed, the resulting mixture was allowed to stand at room temperature, and then the solvent was removed under reduced pressure.

The water from the resulting crude product was removed by azeotropic distillation with toluene and a solid dried under vacuum was obtained.

76.90 g of a white amorphous solid, sufficiently pure to be used in the next reaction stage, were obtained.

The pure product was obtained as a monohydrate by esterification with (-)-menthyl and subsequent basic hydrolysis.

The novel product was characterized by means of melting point, infrared spectrum (IR), ¹H and ¹³C nuclear magnetic resonance (NMR), and mass spectrometry, showing the following results:
- Melting point: > 350° C (decomposition).
- IR (cm⁻¹): 3427; 1610; 1556; 1481; 1425; 1375; 1202; 1156; 1029.
- ¹H-NMR (500 MHz, D₂O): 3.75 (s, 3H), 6.79 (dd, J = 2.4 Hz, J = 8.8 Hz, 1 H), 7.08 (d, J = 2.3 Hz, 1 H), 7.43 (d, J = 8.8 Hz, 1 H).
- ¹³C-NMR (500 MHz, D₂O): 56.2; 99.3; 111.8; 117.6; 137.0; 141.9; 155.2; 167.8.
- Mass spectrometry
   Exact mass: C₈H₇N₂Na₂O₃S (M+H)
   m/z calculated: 256.9967
   m/z found: 256.9964

### Example 2.- Preparation of 5-difluoromethoxy-2-benzimidazolylsulphinic acid disodium salt, compound of general formula (IV) wherein R₂ is 5-difluoromethoxy, and M and R₃ are Na⁺

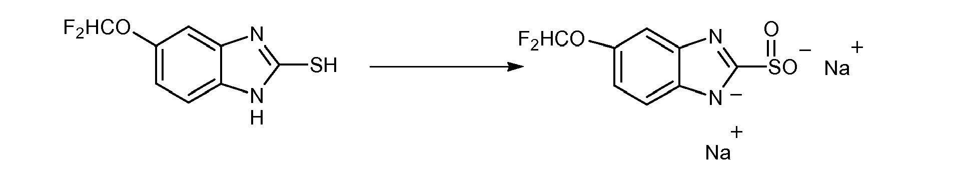

Following the procedure described in Example 1 and using 2-mercapto-5-difluoromethoxybenzimidazole as starting material, 5-difluoromethoxy-2-benzimidazolylsulphinic acid disodium salt was prepared as a white solid.

2-mercapto-5-difluoromethoxybenzimidazole may be prepared in accordance with the process in EP-A-166287.

This product was characterized by means of melting point, infrared spectrum (IR), ¹H and ¹³C nuclear magnetic resonance (NMR), and mass spectrometry, showing the following results:
- Melting point: > 250° C (decomposition).
- IR (cm⁻¹): 3401; 1552; 1485; 1461; 1414; 1361; 1195; 1107; 1044; 1005.
- ¹H-NMR (500 MHz, D₂O): 6.68 (t, J = 74.7 Hz, 3H), 6.97 (dd, J = 2.3 Hz, J = 8.7 Hz, 1 H), 7.33 (d, J = 2.0 Hz, 1 H), 7.51 (d, J = 8.7 Hz, 1 H).
- ¹³C-NMR (500 MHz, D₂O): 107.4; 115.0; 117.2; 120.6; 139.3; 141.6; 146.4; 168.8.
- Mass spectrometry
   Exact mass: C₈H₅F₂N₂Na₂O₃S (M+H)
   m/z calculated: 292.9784
   m/z found: 292.9799

### Example 3.- Preparation of 2-benzimidazolylsulphinic acid disodium salt, compound of general formula (IV) wherein R₂ is hydrogen and M and R₃ are Na⁺

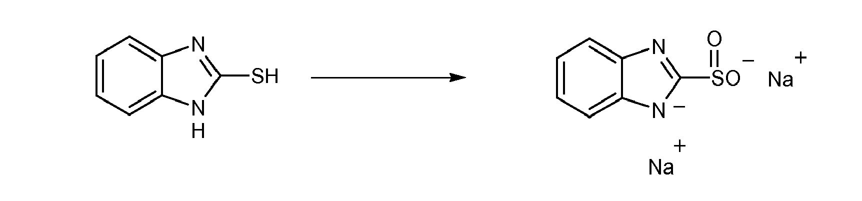

Following the procedure described in Example 1 and using 2-mercaptobenzimidazole as starting material, 2-benzimidazolylsulphinic acid disodium salt was quantitatively prepared as a white solid.

2-mercaptobenzimidazole may be obtained from commercial sources (Fluka).

This product was characterized by means of melting point, infrared spectrum (IR), ¹H and ¹³C nuclear magnetic resonance (NMR), and mass spectrometry, showing the following results:
- Melting point: > 350° C (unmelted).
- IR (cm⁻¹): 3435; 1650; 1455; 1380; 1368; 1275; 1040; 1011; 998.
- ¹H-NMR (500 MHz, D20): 7.10-7.20 (m, 2H); 7.50-7.60 (m, 2H).
- ¹³C-NMR (500 MHz, D20): 117.0; 121.5; 142.8; 169.4.
- Mass spectrometry
   Exact mass: C₇H₅N₂Na₂O₂S (M+H)
   m/z calculated: 226.9873
   m/z found: 226.9867

### Example 4.- Preparation of (-)-menthyl 5-methoxy-2-benzimidazolylsulphinate, compound of general formula (I) wherein R₂ is 5-methoxy, R is O-(-)-menthyl, and R₃ is hydrogen

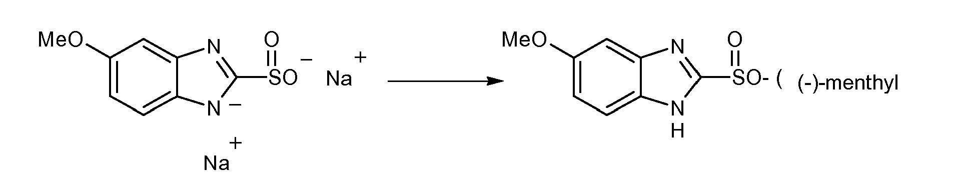

44.8 g of the crude product obtained in Example 1 were suspended in 450 ml of dichloromethane at room temperature.

The mixture was cooled below -5°C, 37.98 g (0.315 mol) of pivaloyl chloride and 3.32 g (0.035 mol) of 4-picoline were added, and then the mixture was stirred at the same temperature for 30 minutes.

Thereafter, 22.1 g (0.14 mol) of (-)-menthol dissolved in 50 ml of dichloromethane were slowly added and the temperature was maintained below 0°C.

Once the addition was completed, the resulting mixture was allowed to stand at room temperature over a period of 1-2 hours approximately.

100 ml of water were added and the aqueous phase was adjusted to a pH 7-9, the organic phase of dichloromethane was separated and the aqueous phase was extracted with dichloromethane (2 x 100 ml).

The combined organic phases were dried over anhydrous magnesium sulphate, filtered and the solvent was removed under reduced pressure.

The obtained crude product was purified by column chromatography (silica gel), using heptane/ethyl acetate (4:1) as eluent.

33.03 g of a white solid were obtained. Total yield was 54% which corresponded to the two steps performed starting from 2-mercapto-5-methoxybenzimidazole.

The novel compound was characterized by means of melting point, infrared spectrum (IR), ¹H and ¹³C nuclear magnetic resonance (NMR), elemental analysis and mass spectrometry, showing the following results:
- Melting point: 138 -140° C.
- IR (cm⁻¹): 2952; 1626; 1589; 1509; 1464; 1406; 1215; 1134; 1119; 1027.
- ¹H-NMR (500 MHz, CDCl₃) (diastereoisomeric mixture 3/2): 0.55-0.95 (m, 10H); 0.95-1.10 (m, 1H); 1.20-1.50 (m, 3H); 1.60-1.75 (m, 2H); 1.95-2.35 (m, 2H); 3.85 (s, 3H); 4.15-4.35 (m, 1 H); 6.90-7.00 (m, 6/5H); 7.03 (dd, J = 2.3 Hz, J = 8.9 Hz, 2/5H); ,7.8 (s, 2/5H); 7.42 (d, J = 8.8 Hz, 2/5H); 7.71 (d, J = 8.5 Hz, 3/5H); 10.44 (s wide, 1 H).
- ¹³C-NMR (500 MHz, CDCl₃) (diastereoisomeric mixture 3/2): 15.5; 15.7; 20.8; 20.9; 21.8; 23.1; 23.2; 25.2; 25.3; 31.8; 31.9; 33.8; 42.9; 47.8; 48.2; 55.8; 82.8; 82.9; 94.3; 102.3; 112.5; 114.0; 116.4; 121.9; 133.6; 158.4.
- Elemental analysis: C₁₈H₂₆N₂O₃S
   Calculated %: C 61.69; H 7.48; N 7.99; S 9.15
   Found %: C 61.67; H 7.18; N 8.05; S 9.12
- Mass spectrometry:
   Exact mass: C₁₈H₂₇N₂O₃S (M+H)
   m/z calculated: 351.1736
   m/z found: 351.1731

### Example 5.- Preparation (-)-fenchyl 5-methoxy-2-benzimidazolylsulphinate, compound of general formula (I) wherein R₂ is 5-methoxy, R is O-(-)-fenchyl, and R₃ is hydrogen

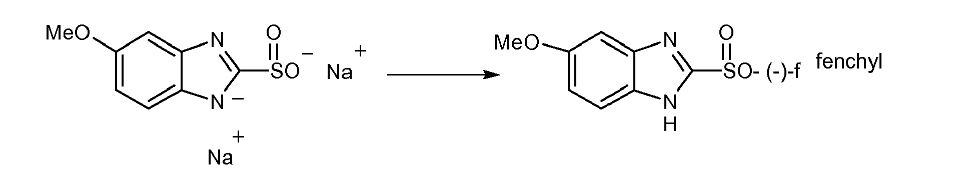

Following a procedure analogous to that described in Example 4 and using the crude product obtained in Example 1 (pivaloyl chloride and (-)-fenchyl((-)-1,3,3-trimethyl-2-norbornanol)alcohol) (-)-fenchyl 5-methoxy-2-benzimidazolyl-sulphinate was prepared.

A white solid was obtained with a total yield of 51% corresponding to the two steps performed starting from 2-mercapto-5-methoxybenzimidazole.

The novel compound was characterized by means of melting point, infrared spectrum (IR), ¹H and ¹³C nuclear magnetic resonance (NMR), elemental analysis and mass spectrometry, showing the following results:
- Melting point: 121-123° C.
- IR (cm⁻¹): 2961; 1624; 1588; 1508; 1462; 1396; 1308; 1204; 1176; 1133; 1118; 1035.
- ¹H-NMR (500 MHz, CDCl₃) (diastereoisomeric mixture 3/2): 0.60-1.20 (m, 11 H); 1.35-1.50 (m, 2H); 1.60-1.75 (m, 3H); 3.82 (d, J = 1.8 Hz, 3/5H); 3.86 (s, 3H); 3.94 (d, J = 1.6 Hz, 2/5H); 6.95-7.00 (m, 6/5H); 7.00-7.05 (m, 2/5H); 7.25-7.30 (m, 2/5H); 7.40-7.50 (m, 2/5H); 7.65-7.75 (m, 3/5H); 10.60 (s wide, 3/5H); 10.65 (s wide, 2/5H).
- ¹³C-NMR (500 MHz, CDCl₃) (diastereoisomeric mixture 3/2): 18.8; 19.2; 21.4; 21.6; 25.7; 25.8; 25.9; 29.2; 29.5; 39.6; 39.7; 41.1; 41.2; 47.8; 48.0; 49.1; 49.3; 55.7; 55.8; 93.3; 93.7; 93.9; 94.2; 102.1; 112.5; 114.1; 116.4; 121.9; 133.6; 138.3; 144.7; 156.8; 158.4.
- Elemental analysis: C₁₈H₂₄N₂O₃S
   Calculated %: C 62.04; H 6.94; N 8.04; S 9.20
   Found %: C 62.31; H 6.68; N 8.01; S 9.20
- Mass spectrometry:
   Exact mass: C₁₈H₂₅N₂O₃S (M+H)
   m/z calculated: 349.1580
   m/z found: 349.1581

### Example 6.- Preparation (-)-menthyl 5-difluoromethoxy-2-benzimidazolylsulphinate, compound of general formula (I) wherein R₂ is 5-difluoromethoxy, R is O-(-)-menthyl, and R₃ is hydrogen

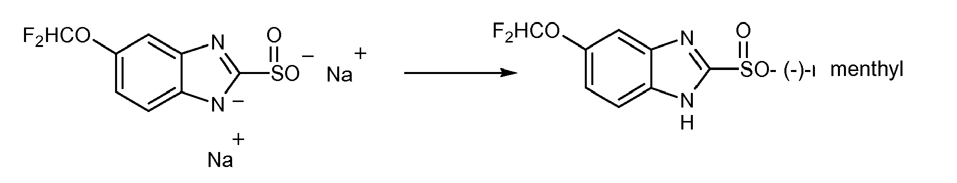

Following a procedure analogous to that described in Example 4 and using the crude product obtained in Example 2 (pivaloyl chloride and (-)-menthol), (-)-menthyl5-difluoromethoxy-2-benzimidazolylsulphinate was prepared.

A colourless oil was obtained with a total yield of 48% corresponding to the two steps performed starting from 2-mercapto-5-difluoromethoxybenzimidazole.

The novel compound was characterized by means of melting point, infrared spectrum (IR), ¹H and ¹³C nuclear magnetic resonance (NMR) and mass spectrometry, showing the following results:
- IR (cm-1): 2958; 1627; 1456; 1384; 1180; 1129; 1048; 945; 907.
- ¹H-NMR (500 MHz, CDCl₃) (diastereoisomeric mixture 5/4): 0.55-1.10 (m, 11 H); 1.20-1.50 (m, 3H); 1.60-1.75 (m, 2H); 1.95-2.35 (m, 2H); 4.20-4.29 (m, 5/9H); 4.29-4.37 (m, 4/9H); 6.52 (dt, J = 1.35 Hz, J = 73.85 Hz, 1 H); 7.16 (d, J = 8.4 Hz, 1 H), 7.46 (s wide, 1 H); 7.69 (s wide, 1 H); 11.25 (s wide, 1 H).
- ¹³C-NMR (500 MHz, CDCl₃) (diastereoisomeric mixture 5/4): 15.47; 15.65; 20.73; 20.78; 21.78; 21.79; 23.09; 23.12; 25.27; 25.33; 31.81; 31.88; 33.70; 33.73; 42.83; 42.95; 47.78; 48.15; 83.61; 83.82; 114.06; 116.14; 118.20.
- Mass spectrometry:
   Exact mass: C₁₈H₂₅F₂N₂O₃S (M+H)
   m/z calculated: 387.1548
   m/z found: 387.1555

### Example 7.- Preparation of (-)-menthyl 2-benzimidazolylsulphinate, compound of general formula (I) wherein R₂ is hydrogen, R is O-(-)-menthyl, and R₃ is hydrogen

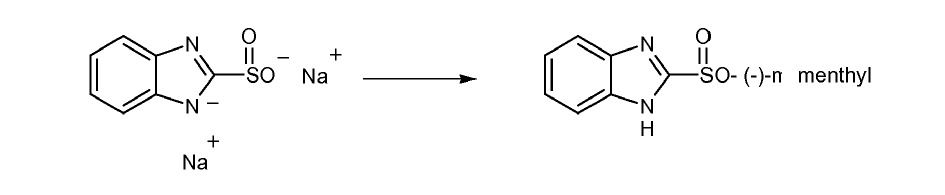

Following a procedure analogous to that described in Example 4 and using the crude product obtained in Example 3 (pivaloyl chloride and (-)-menthol), (-)-menthyl 2-benzimidazolylsulphinate was prepared.

A white solid was obtained with a total yield of 58% corresponding to the two steps performed starting from 2-mercaptobenzimidazole.

The novel compound was characterized by means of melting point, infrared spectrum (IR), ¹H and ¹³C nuclear magnetic resonance (NMR), elemental analysis and mass spectrometry, showing the following results:
- Melting point: 129-130° C.
- IR (cm⁻¹): 2960; 1432; 1414; 1300; 1272; 1134; 1010; 944; 907; 847.
- ¹H-NMR (500 MHz, CDCl₃) (diastereoisomeric mixture 5/4): 0.55-0.90 (m, 10H); 0.90-1.10 (m, 1H); 1.20-1.50 (m, 3H); 1.60-1.75 (m, 2H); 1.95-2.35 (m, 2H); 4.20-4.27 (m, 4/9H); 4.27-4.35 (m, 5/9H); 7.30-7.40 (m, 2H); 7.56 (d, J = 7.9 Hz, 1 H); 7.85 (d, J = 7.9 Hz, 1 H); 10.71 (s wide, 1H).
- ¹³C-NMR (500 MHz, CDCl₃) (diastereoisomeric mixture 5/4): 15.5; 15.7; 20.7; 20.8; 21.8; 23.0; 23.1; 25.2; 25.3; 31.8; 31.9; 33.7; 42.8; 42.9; 47.7; 48.1; 83.1; 83.3; 112.1; 121.2; 121.3; 123.5; 125.3; 132.8; 143.8; 153.8.
- Elemental analysis: C₁₇H₂₄N₂O₂S
   Calculated %: C 63.72; H 7.55; N 8.74; S 10.01
   Found %: C 63.80; H 7.18; N 8.70; S 10.16
- Mass spectrometry:
   Exact mass: C₁₇H₂₅N₂O₂S (M+H)
   m/z calculated: 321.1631
   m/z found: 321.1624

### Example 8.- Preparation of ethyl 2-benzimidazolylsulphinate, compound of general formula (I) wherein R₂ is hydrogen, R is ethoxy, and R₃ is hydrogen

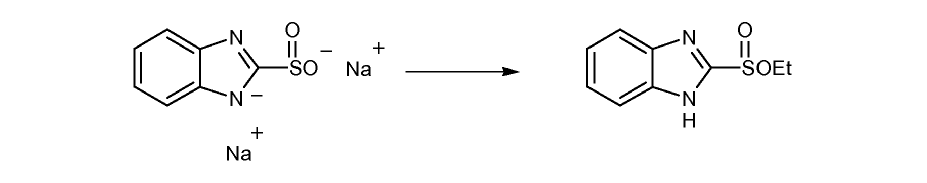

Following a procedure analogous to that described in Example 4 and using the crude product obtained in Example 3 (pivaloyl chloride and ethanol), ethyl 2-benzimidazolylsulphinate was prepared.

A white solid was obtained.

The novel compound was characterized by means of melting point, infrared spectrum (IR), ¹H and ¹³C nuclear magnetic resonance (NMR) and mass spectrometry, showing the following results:
- Melting point: 148-149° C.
- IR (cm⁻¹): 2980; 1472; 1430; 1300; 1273; 1142; 996; 979; 896.
- ¹H-NMR (500 MHz, CDCl₃): 1,32 (t, J = 7.0 Hz, 3H); 3.80-3.90 (m, 1 H); 4.25-4.35 (m, 1 H); 7.37 (s wide, 1 H); 7.58 (s wide, 1 H); 7.86 (s wide, 1 H); 10.98 (s wide, 1 H).
- ¹³C-NMR (500 MHz, CDCl₃): 15.4; 63.4; 110.0; 112.2; 121.3; 123.5; 123.7; 125.4; 152.8.
- Mass spectrometry:
   Exact mass: C₉H₁₁N₂O₂S (M+H)
   m/z calculated: 211.0535
   m/z found: 211.0534

### Example 9.- Preparation of omeprazole, compound of general formula (II) wherein R ₂ is 5-methoxy, R₄ and R ₆ are methyl, and R₅ is methoxy

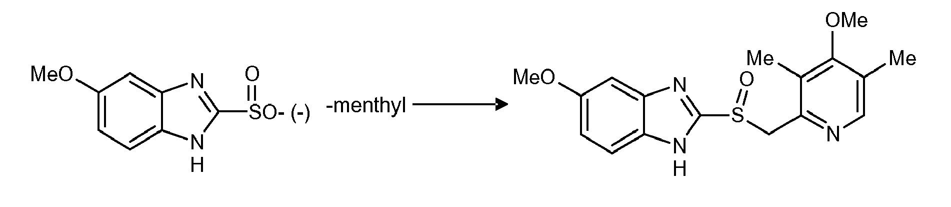

1.0 g (6.61 mmol) of 4-methoxy-2,3,5-trimethylpiridine were dissolved in 10 ml of anhydrous tetrahydrofuran at room temperature under inert atmosphere, and the solution obtained was cooled to below -90° C.

4-methoxy-2,3,5-trimethylpiridine may be prepared in accordance with the process described in EP-A-0005129.

Thereafter, 2.6 ml (6.5 mmol) of a 2.5 M solution of *n*-butyl lithium was added dropwise and the temperature was maintained below - 80°C. After 30 minutes at this temperature, the mixture was slowly added to a solution of 0.65 g (1.86 mmol) of (-)-menthyl 5-methoxy-2-benzimidazolylsulphinate, as prepared in Example 4, in 3.7 ml of tetrahydrofuran cooled below -80°C as well.

Once the addition was completed, the resulting mixture was allowed to stand up to -20° C, then 20 ml of water were slowly added and it was allowed to reach room temperature.

The reaction mixture was analyzed by HPLC, and conversion and yield were found to be 99% and 86% respectively.

### Example 10.- Preparation of rabeprazole, compound of general formula (11) wherein R₂ is hydrogen, R₄ is hydrogen, R₅ is methyloxypropyloxy, and R₆ is methyl

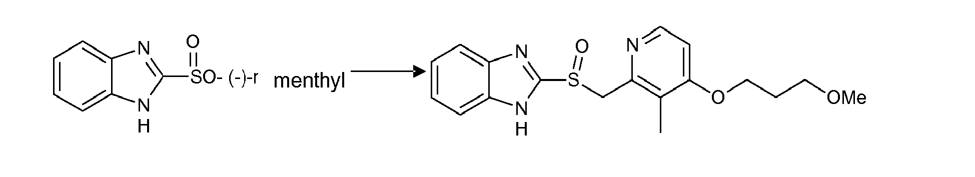

4.26 g (21.8 mmol) of 2,3-dimethyl-4-(3-methoxy-propoxy)piridine were dissolved in 42 ml of anhydrous tetrahydrofuran at room temperature under inert atmosphere, and the solution was then cooled below - 90° C. 8.72 ml of a 2.5M solution of *n*-butyl lithium (21.8 mmol) were added dropwise and the temperature was maintained below -80° C.

2,3-dimethyl-4-(3-methoxy-propoxy)pyridine may be prepared in accordance with the process described in EP-A-0268956.

After 30 minutes at this temperature, the mixture was slowly added to a solution of 2.0 g (6.24 mmol) of (-)-menthyl 2-benzimidazolylsulphinate, as obtained in Example 7, in 36 ml of tetrahydrofuran cooled below -80°C as well.

Once the addition was completed, the resulting mixture was allowed to stand up to -20° C, then 100 ml of water were slowly added and it was allowed to reach room temperature.

The reaction mixture was analyzed by HPLC, and conversion and yield were found to be 95% and 90% respectively.

### Example 11.- Separation of (-)-menthyl 5 -methoxy-2-benzimidazolylsulphinate diastereoisomers

For separation of the two diastereoisomers, an analytical column (Discovery ZR-CARBON 15 cm x 4.6 mm, 5µm) supplied by SUPELCO was used, employing the following chromatographic conditions:

| | |
|---|---|
| Temperature | 50°C |
| Flow rate | 3 ml/min |
| Detection | 302 nm |
| Injection volume | 100 µl |
| Concentration of sample | 5 g/l |
| Analysis time | 30 min |

Solvent consisted of a mixture of 10% water, 80% acetonitrile, and 10% tetrahydrofuran (10:80:10, v/v/v).

Twenty-five injections were made and the fractions were collected at the following times:
- F1: from 9.20 to 13.50 minutes
- F2: from 13.80 to 23.00 minutes

Both fractions, F1 and F2 , were concentrated under vacuum separately, and about 6 mg of product were recovered in each of the fractions.

### Example 12.- Preparation of esomeprazole, S-isomer of the compound of general formula (II) wherein R₂ is 5-methoxy, R₄ and R₆ are methyl, and R₅ is methoxy

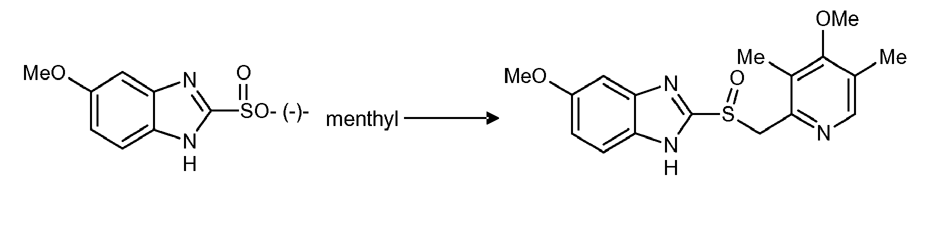

26 mg (0.17 mmol) of 4 -methoxy-2,3,5-trimethylpiridine were dissolved in 0.5 ml of anhydrous tetrah ydrofuran at room temperature under under inert atmosphere and the solution was then cooled below -90°C.

0.10 ml of a 1.7M solution of terc-butyl lithium (0.17 mmol) were added dropwise and the temperature was maintained below -80°C.

After 30 minutes at this temperature, the mixture was slowly added to a solution 6 mg (0.017 mmol) of the F1 isomer of (-)-menthyl 5-methoxy-2-benzimidazolylsulphinate, as obtained in Example 11, in 1 ml de tetrahydrofuran cooled below -80°C as well.

Once the addition was completed, the resulting mixture was allowed to stand up to -20° C, then 5 ml of water were slowly added and it was allowed to reach room temperature.

The reaction mixture was analyzed by HPLC and 92% conversion occurred.

## Claims

1. A process for preparing compounds of general formula (II) 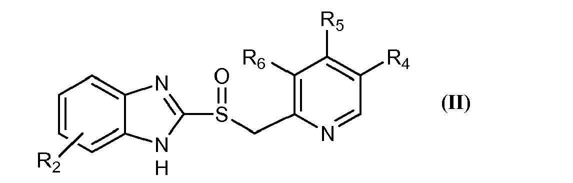 wherein:
R₂ is hydrogen, C₁-C₄ alkyl, C₁-C₄ alkoxy, C₁-C₄ alkoxy partially or totally substituted with halogen atoms,
R₄, R₅ and R₆, independently represent hydrogen, C₁-C₈ alkyl, C₁-C₈ alkoxy, C₁-C₈ alkoxy partially or totally substituted with alkoxide groups, C₁-C₈ alkoxy partially or totally substituted with halogen atoms,
**characterized in that** it comprises the reaction of a compound of general formula (I): 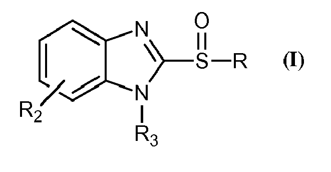 wherein:
R₂ is hydrogen, C₁-C₄ alkyl, C₁-C₄ alkoxy, C₁-C₄ al koxy partially or totally substituted with halogen atoms, and
R is -OR₁, wherein R₁ is C₁-C₈ alkyl, C₂-C₈ alkenyl, C₃-C₂₀, cycloalkyl or C₇-C₂₀ alkylaryl,
R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, carbonyloxyalkyl or silylalkyl,
with a metalated derivative of the compound of formula (III): 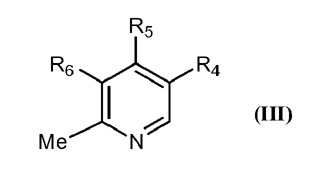 wherein:
R₄, R₅ and R₆, independently represent hydrogen, C₁-C₈ alkyl, C₁-C₈ alkoxy, C₁-C₈ alkoxy partially or totally substituted with alkoxide groups, C₁-C₈ alkoxy partially or totally substituted with halogen atoms,
in the core of an inert solvent.

2. The process according to claim 1, **characterized in that** the compounds have the general formula (II) wherein R₂ is hydrogen, C₁-C₃ alkoxy or C ₁-C₃ alkoxy totally or partially substituted with fluor atoms.

3. The process according to claim 2, **characterized in that** R₂ is hydrogen, methoxy, or difluoromethoxy.

4. The process according to claim 3, **characterized in that** the compound of general formula (II) is omeprazole, pantoprazole, lansoprazole or rabeprazole.

5. The process according to any one of claims 1 to 4, **characterized in that** the compounds have the general formula (I) wherein R₂ is hydrogen, C₁-C₃ alkoxy or C₁-C₃ alkoxy totally or partially substituted with fluor atoms, R is -OR₁ wherein R₁ is C₁-C₄ alkyl, C₃-C₁₂ cycloalkyl, or C₇-C₁₆, alkylaryl and R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl.

6. The process according to claim 5, **characterized in that** R₂ is hydrogen, methoxy, or difluoromet hoxy, and R is -OR₁ wherein R₁ is methyl, ethyl, *n-*propyl, *i*-propyl, (-)-menthyl, (+)-menthyl, (-)-fenchyl, (+)-fenchyl, (-)-8-phenylmenthyl, (+)-8-phenylmenthyl, and R₃ is hydrogen, or alkali or alkaline earth metal cation.

7. The process according to claim 6, **characterized in that** R₂ is hydrogen, methoxy, or difluoromethoxy, and R is -OR₁ wherein R₁ is ethyl, (-)-menthyl, or (-)-fenchyl.

8. The process according to any one of claims 1 to 7, **characterized in that** the metalated derivative of compound (III) is a compound of formula (VI) 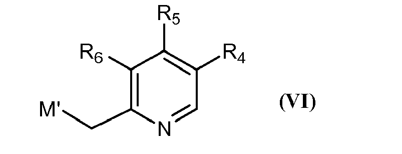 wherein:
R₄, R₅ and R₆ are as defined above, and
M' is an alkali or alkaline earth metal cation, a Mg-halogen cation, or a Zn-halogen cation.

9. The process according to claim 8, **characterized in that** M' is a lithium cation.

10. The process according to any one of claims 1 to 9, **characterized in that** the compound of formula (III) is selected from the group consisting of the compounds wherein R₄ is hydrogen or methyl, R₅ is methoxy, methyloxypropyloxy, or 2,2,2-trifluoroethoxy, and R₆ is methyl or methoxy.

11. The process according to claim 10, **characterized in that** R₄ is hydrogen and R₅ and R₆ are methoxy; R₄ is hydrogen, R₅ is methyloxypropyloxy and R₆ is methyl; R₄ is methyl, R₅ is methoxy and R₆ is methyl; and R₄ is hydrogen, R₅ is 2,2,2-trifluoroehtoxy, and R₆ is methyl.

12. The process according to claim 1, **characterized by** comprising a previous step that comprises the separation of the optical isomers of the compound of general formula (I) wherein R₁ is a radical corresponding to a chiral alcohol.

13. The process according to claim 12, **characterized in that** the chiral alcohol is selected from the group consisting of (-)-menthol, (+)-menthol, (-)-fenchol, (+)-fenchol, (-)-8-phenylmenthol, and (+)-8-phenylmenthol.

14. The process according to claim 13, **characterized in that** the chiral alcohol is (-)-menthol, or (-)-fenchol.

15. The process according to any one of claims 12 to 14, **characterized in that** the compound of general formula (II) is S-omeprazole, R-omeprazole, S-pantoprazole, R-pantoprazole, S-lansoprazole, R-lansoprazole, S-rabeprazole, or R-rabeprazole.

16. A process for the preparation of compounds of general formula (I) 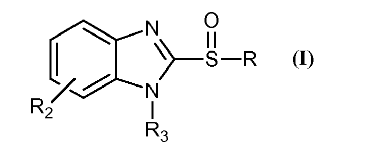 wherein :
R₂ is hydrogen, C₁-C₄ alkyl, C₁-C₄ alkoxy, C₁-C₄ alkoxy partially or totally substituted with halogen atoms, and
R is -OR₁, wherein R₁ is C₁-C₈ alkyl, C₂-C₈ alkenyl, C₃-C₂₀ cycloalkyl or C₇-C₂₀ alkylaryl,
R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl,
**characterized in that** it comprises reacting a compound of general formula (IV) 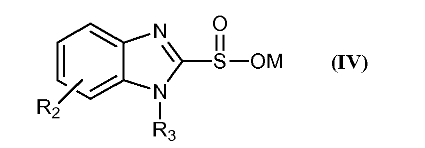 wherein:
R₂ is hydrogen, C₁-C₄ alkyl, C₁-C₄ alkoxy, C₁-C₄ alkoxy partially or totally substituted with halogen atoms,
M is an alkali or alkaline earth metal cation, and
R₃ is hydrogen, alkali or alkaline earth metal cation, C₁-C₄, alkyl, C₁-C₄ alkyl partially or totally substituted with halogen atoms, alkenyl, sulfonylalkyl, sulfonylamino, carbonylalkyl, cabonyloxyalkyl or silylalkyl,
with a C₁-C₈ alkyl, C₂-C₈ alkenyl, C₃-C₂₀ cycloalkyl or C₇-C₂₀ alkylaryl alcohol by previously converting the -OM group of compound (IV) into a leaving group.

17. The process according to claim 16, **characterized by** obtaining a compound of general formula (I) wherein R₂ is hydrogen, C₁-C₃ alkoxy or C₁-C₃ alkoxy partially or totally substituted with fluor atoms, R is -OR₁ wherein R₁ is C₁-C₄ alkyl, C₃-C₁₂ cycloalkyl, or C₇-C₁₆, alkylaryl and R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl.

18. The process according to claim 17, **characterized by** obtaining a compound of general formula (I) wherein R₂ is hydrogen, methoxy, or difluoromethoxy, and R is -OR₁ wherein R₁ is methyl, ethyl, *n*-propyl, *i*-propyl, (-)-menthyl, (+)-menthyl, (-)-fenchyl, (+)-fenchyl, (-)-8-phenylmenthyl, (+)-8-phenylmenthyl, and R₃ is hydrogen.

19. The process according to claim 18, **characterized by** obtaining a compound of general formula (I) wherein R₂ is hydrogen, methoxy, or difluoromethoxy, R is -OR₁, wherein R₁ is ethyl, (-)-menthyl, or (-)-fenchyl.

20. The process according to any one of claims 16 to 19, **characterized in that** the starting material is a compound of general formula (IV) wherein R₂ is hydrogen, C₁-C₃ alkoxy or C₁-C₃ alkoxy totally or partially substituted with fluor atoms, R₃ is hydrogen or an alkali or alkaline earth metal cation, C₁-C₄ alkyl, C₁-C₄ alkyl partially or totally substituted with halogen atoms, alkenyl, sulfonylalkyl, sulfonylamino, carbonylalkyl, carbonyloxyalkyl or silylalkyl, and M is an alkali or alkaline earth metal cation.

21. The process according to claim 20, **characterized in that** the starting material is a compound of general formula (IV) wherein R₂ is hydrogen, C₁-C₃ alkoxy or C₁-C₃ alkoxy totally or partially substituted with fluor atoms, and R ₃ and M are an alkali or alkaline earth metal cation.

22. The process according to claim 21, **characterized in that** the starting material is a compound of general formula (IV) wherein R₂ is hydrogen, methoxy, or difluoromethoxy, and R₃ and M are a sodium or potassium cation.

23. The process according to any one of claims 16 to 22, **characterized in that** the -OM group of the compound of general formula (IV) is converted into a leaving group by reaction of this compound with a compound selected from the group consisting of oxazolidinones of phosphinic acid halides, alkoxycarbonyl halides, carboxylic acid halides, alkylcarbodiimides or N,N'-carbonyldiimidazoles.

24. The process according to claim 23, **characterized in that** the compound of general formula (IV) is reacted with a C₂-C₆ carboxylic acid chloride.

25. The process according to claim 24, **characterized in that** the compound of general formula (IV) is reacted with pivalic acid chloride.

26. The process according to any one of claims 16 a 25, **characterized by** using a solvent selected from the group consisting of toluene, acetonitrile, dichloromethane, and chloroform.

27. The process according to claim 26, **characterized by** using dichloromethane.

28. The process according to any one of claims 16 to 27, **characterized in that** the alcohol is a C₁-C₈ alkyl, C₂-C₈ alkenyl, C₃-C₂₀ cycloalkyl, or C₇-C₂₀ alkylaryl alcohol.

29. The process according to claim 28, **characterized in that** the alcohol is a C₁-C₄ alkyl, C₃-C₁₂ cycloalkyl or C₇-C₁₆ alkylaryl alcohol.

30. The process according to claim 29, **characterized in that** the alcohol is methanol, ethanol, *n*-propanol, *i*-propanol, (-)-menthol, (+)-menthol, (-)-fenchol, (+)-fenchol, (-)-8-phenylmenthol or (+)-8-phenylmenthol.

31. The process according to any one of claims 16 to 30, **characterized in that** the compound of general formula (IV) 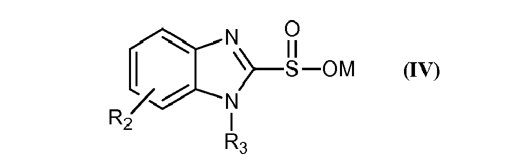 wherein:
R₂ is hydrogen, C₁-C₄ alkyl, C₁-C₄ alkoxy, C₁-C₄ alkoxy partially or totally substituted with halogen atoms,
M is hydrogen, or an alkali or alkaline earth metal cation, and
R₃ is hydrogen, an alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl,
is obtained by a process which consists in reacting a compound of general formula (V): 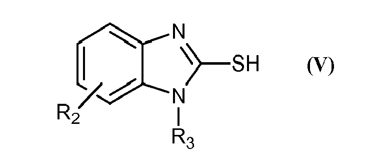 wherein:
R₂ is hydrogen, C₁-C₄ alkyl. C₁-C₄ alkoxy, C₁-C₄ alkoxy partially or totally substituted with halogen atoms, and
R₃ is hydrogen, C₁-C₄ alkyl, C₁-C₄ alkyl partially or totally substituted with halogen atoms, alkenyl, sulfonylalkyl, sulfonylamino, carbonylalkyl, cabonyloyialkyl or silylalkyl,
with an oxidative agent in an alkaline medium.

32. The process according to claim 31, **characterized in that** the obtained product is a compound of general formula (IV) wherein R₂ is hydrogen, C₁-C₃ alkoxy, or C₁-C₃ alkoxy totally or partially substituted with fluor atoms, M is hydrogen or an alkali or alkaline earth metal cation, and R₃ is hydrogen or an alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl.

33. The process according to claim 32, **characterized in that** the obtained product is a compound of general formula (IV) wherein R₂ is hydrogen, C₁-C₃ alkoxy or C₁-C₃ alkoxy totally or partially substituted with fluor atoms, and R ₃ and M are an alkali or alkaline earth metal cation.

34. The process according to claim 33, **characterized in that** the obtained product is a compound of general formula (IV) wherein R₂ is hydrogen, methoxy, or difluoromethoxy, and R₃ and M are a sodium or potassium cation.

35. The process according to any one of claims 31 to 34, **characterized in that** oxidation is performed in the presence of peracids, alky I hydroperoxides, or hydrogen peroxide and catalysis of molybdate salts.

36. The process according to claim 35, **characterized in that** oxidation is performed in the presence of hydrogen peroxide and catalysis of molybdate salts.

37. The process according to claim 36, **characterized in that** oxidation is performed in the presence of hydrogen peroxide and ammonium heptamolybdate.

38. The process according to any one of claims 31 to 37, **characterized in that** the oxidation reaction is carried out in the core of an aqueous solution of an alkali or alkaline earth hydroxide.

39. The process according to claim 38, **characterized in that** the alkali hydroxide is sodium hydroxide.

40. Intermediate compounds of general formula (1) 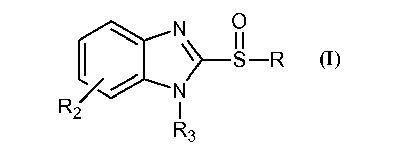 wherein:
R₂ is hydrogen, C₁-C₄ alkyl, C₁-C₄ alkoxy, C₁-C₄ alkoxy partially or totally substituted with halogen atoms, and
R is -OR₁, wherein R₁ is hydrogen, an alkali or alkaline earth metal cation, C₁-C₈ alkyl, C₂-C₈ alkenyl, C₃-C₂₀ cycloalkyl or C₇-C₂₀ alkylaryl,
R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxialkyl or silylalkyl,
and any of their optical isomers, with the exception of the compound wherein R₁, R₂, and R₃ are hydrogen.

41. A compound according to claim 40, **characterized in that** wherein R₂ is hydrogen, C₁-C₃ alkoxy or C₁-C₃ alkoxy totally or partially suibstituted with fluor atoms, R is -OR₁ wherein R₁ is C₁-C₄ alkyl, C₃-C₁₂ cycloalkyl or C₇-C₁₆ alkylaryl and R₃ is hydrogen, alkali or alkaline earth metal cation, C₁-C₄ alkyl, C₁-C₄ alkyl partially or totally substituted with halogen atoms, alkenyl, sulfonylalkyl, sulfonylamino, carbonylalkyl, cabonyloxyalkyl or silylalkyl.

42. A compound according to claim 41, **characterized in that** R₂ is hydrogen, methoxy, or difluoromethoxy, R₁ is methyl, ethyl, *n*-propyl, *i*-propyl, (-)-menthyl, (+)-menthyl, (-)-fenchil, (+)-fenchil, (-)-8-phenylmenthyl, (+)-8-phenylmenthyl, and R₃ is hydrogen.

43. A compound according to claim 42, **characterized in that** R₁ is ethyl, (-)-menthyl, or (-)-fenchil.

44. A compound according to claim 40, **characterized in that** R₂ is hydrogen, C₁-C₃ alkoxy or C₁-C₃ alkoxy totally or partially substituted with fluor atoms, R is -OR₁ wherein R₁ is an alkali or alkaline earth metal cation and R₃ is hydrogen or an alkali or alkaline earth metal cation.

45. A compound according to claim 44, **characterized in that** R₂ is hydrogen, methoxy, or difluoromethoxy, and R₁ and R₃ are an alkali metal cation.

46. A compound according to claim 45, **characterized in that** R₁ and R₃ are a sodium or potassium cation.

47. Use of 2-benzimidazolylsulphinic acid derivatives of general formula (I) for the preparation of 2-(2-pyridylmethyl)sulphinyl-1H-benzimidazoles of general formula (II) and their pharmaceutically acceptable salts.

48. The use according to claim 47, **characterized in that** the 2-benzimidazolylsulphinic acid derivatives are any one of the compounds in claims 41 to 43.

49. The use according to claim 47, **characterized in that** 2-benzimidazolylsulphinic acid derivatives are any one of the compounds in claims 44 to 46.

50. The use according to any one fo claims 47 to 49, **characterized by** preparing omeprazole, pantoprazole, lansoprazole, rabeprazole, or esomeprazole.
